# FASCICULE DE BREVET EUROPEEN

(11) **EP 3 554 428 B1**
(45) Date de publication et mention de la délivrance du brevet: **23.12.2020**
(21) Numéro de dépôt: 17821989.5
(22) Date de dépôt: 13.12.2017
(51) Int. Cl.: A61F 2/46, A61F 2/30

(54) **ANCILLAIRE DE POSE D'UNE CUPULE POUR IMPLANT COTYLOÏDIEN DE PROTHÈSE TOTALE DE HANCHE**
HILFSVORRICHTUNG ZUM IMPLANTIEREN EINER PFANNE FÜR EIN HÜFTGELENKPFANNENIMPLANTAT EINER TOTALHÜFTPROTHESE
ANCILLARY DEVICE FOR IMPLANTING A CUP FOR A COTYLOID IMPLANT OF A TOTAL HIP PROSTHESIS

(30) Priorité: 13.12.2016 FR 1662378
(43) Date de publication de la demande: 23.10.2019
(73) Titulaire: Amplitude, 26000 Valence (FR); DM 2, 45560 Saint-Denis-en-Val (FR)
(72) Inventeur: DELALANDE, Jean-Luc Christian Jules, 45560 Saint-Denis-en-Val (FR); DENJEAN, Stéphane, 71960 La Roche Vineuse (FR); GAILLARD, Thierry Alain Michel, 69003 Lyon (FR); TAYOT, Olivier Louis, 69300 Caluire-Et-Cuire (FR); RIMET, Laëtitia, 26300 Bourg de Peage (FR); CHAVANE, Hervé, 69300 Caluire et Cuire (FR); DOMB, Benjamin, Glencoe, IL 60022 (US); CHEVILLOTTE, Christophe Jean François Marie, 71640 Givry (FR)
(74) Mandataire: Chevalier, Renaud Philippe
(86) Numéro de dépôt international: PCT/FR2017/053546
(87) Numéro de publication internationale: WO 2018/109379

(56) Documents cités:
- WO-A1-2009/136284
- WO-A1-2016/055851
- US-A1- 2016 135 963
- US-B1- 8 398 650

## Description

La présente invention se rapporte à un ancillaire de pose d'une cupule pour implant cotyloïdien de prothèse totale de hanche.

Elle se rapporte plus particulièrement à un ancillaire comportant :
- un manche de manipulation comprenant, d'une part, une partie distale conçue pour une saisie manuelle et présentant une surface d'impaction et, d'autre part, une partie proximale incurvée se terminant par une tête de préhension ;
- un organe de préhension propre à coopérer avec la tête de préhension pour la préhension de la cupule ; et
- un système d'actionnement comprenant une poignée mobile montée pivotante selon un axe de pivotement sur le manche et un dispositif de transmission mécanique reliant ladite poignée audit organe de préhension, ledit dispositif de transmission mécanique comprenant une biellette incurvée guidée en déplacement par la partie proximale du manche et traversant la tête de préhension pour coopérer avec ledit organe de préhension ;
où, pour déplacer l'organe de préhension, la poignée est actionnable entre :
- une position déployée de manière à ce que l'organe de préhension soit éloigné de la tête de préhension pour permettre un déverrouillage de la cupule sur l'organe de préhension ; et
- une position rabattue de manière à ce que l'organe de préhension coopère avec la tête de préhension pour permettre un verrouillage de la cupule sur l'organe de préhension.

De manière connue, la poignée est articulée sur le manche sans possibilité de démontage de la poignée aisée et rapide, ce qui représente un inconvénient pour les opérations ultérieures de nettoyage et de stérilisation, sans compter que cette absence de possibilité de démontage de la poignée permet une utilisation de l'ancillaire pour un seul type de cupule.

Pour résoudre au moins en partie ce problème, il est connu du document WO 2016/055851 de prévoir un ancillaire dont la poignée est montée pivotante et de manière amovible sur le manche, autorisant le retrait de la poignée pour des opérations de nettoyage et de stérilisation. A cet effet, la poignée présente, à une première extrémité articulée sur le manche, deux pions cylindriques et saillants réceptionnés dans des encoches évasées respectives prévues dans le manche. Cependant, la forme des ergots et des encoches présentent l'inconvénient que la poignée n'est pas maintenue stablement dans les encoches, créant ainsi des risques de désolidarisation de la poignée non voulue ainsi que des mouvements non désirés de la poignée qui peuvent gêner lors de la manipulation par le chirurgien.

Il est également connu du document US 2016/135963 de prévoir un ancillaire dont la poignée est montée pivotante et de manière amovible sur le manche, autorisant le retrait de la poignée. A cet effet, le manche présente une fente recevant intérieurement un axe de pivot fixe, et la poignée présente une extrémité munie d'une encoche montée pivotante sur l'axe de pivot, et la poignée comprend en outre un verrou qui verrouille l'encoche de la poignée sur l'axe de pivot. L'emploi du verrou présente l'avantage de pouvoir verrouiller la poignée sur l'axe de pivot, évitant ainsi une désolidarisation de la poignée non voulue, mais cependant ce verrou s'avère complexe et donc peu pratique pour l'utilisateur.

La présente invention a pour but de résoudre en tout ou partie les inconvénients précités en proposant un ancillaire qui permet un non démontage de la poignée lors de l'utilisation normale de l'ancillaire, en utilisant des moyens simples, peu coûteux et non contraignants pour l'utilisateur.

Un autre but de l'invention est de permettre au chirurgien de ressentir, durant la course de basculement de la poignée, le stade de coopération entre la cupule et l'organe de préhension.

Un autre but de l'invention est de permettre une utilisation d'un même manche avec plusieurs types de cupules, en remplaçant uniquement tout ou partie du dispositif de transmission mécanique pour qu'il soit adapté à l'organe de préhension qui convient à la cupule concernée.

A cet effet, elle propose un ancillaire de pose d'une cupule pour implant cotyloïdien de prothèse totale de hanche, du type comportant :
- un manche de manipulation comprenant, d'une part, une partie distale conçue pour une saisie manuelle et présentant une surface d'impaction et, d'autre part, une partie proximale incurvée se terminant par une tête de préhension ;
- un organe de préhension propre à coopérer avec la tête de préhension pour la préhension de la cupule ; et
- un système d'actionnement comprenant une poignée mobile montée pivotante selon un axe de pivotement sur le manche et un dispositif de transmission mécanique reliant ladite poignée audit organe de préhension, ledit dispositif de transmission mécanique comprenant une biellette incurvée guidée en déplacement par la partie proximale du manche et traversant la tête de préhension pour coopérer avec ledit organe de préhension ;
où, pour déplacer l'organe de préhension, la poignée est actionnable entre :
- une position déployée de manière à ce que l'organe de préhension soit éloigné de la tête de préhension pour permettre un déverrouillage de la cupule sur l'organe de préhension ; et
- une position rabattue de manière à ce que l'organe de préhension coopère avec la tête de préhension pour permettre un verrouillage de la cupule sur l'organe de préhension ;
où la poignée est montée pivotante et de manière amovible sur le manche et l'organe de préhension est fixé de manière amovible sur la biellette incurvée, afin de permettre le démontage de la poignée et du système d'actionnement hors du manche,
où le manche présente une bordure inférieure et une bordure supérieure, et la poignée est articulée sur une partie échancrée de ladite bordure inférieure, ladite partie échancrée étant délimitée par deux parois en vis-à-vis, où les deux parois présentent des encoches en regard l'une de l'autre, et la poignée présente, à une première extrémité articulée sur le manche, deux ergots saillants réceptionnés dans les encoches respectives, de sorte que, d'une part, lesdits ergots puissent pivoter à l'intérieur desdites encoches et, d'autre part, lesdits ergots puissent être retirés hors desdites encoches lors du démontage de la poignée et du système d'actionnement hors du manche,
et où cet ancillaire est remarquable en ce que chaque ergot présente une paroi périphérique cylindrique tronquée sur deux côtés opposés pour définir deux faces planes opposées et deux faces arquées opposées, et chaque encoche est délimitée par une paroi périphérique interne qui présente un fond arqué et deux faces planes en regard formant une entrée rétrécie à l'intérieur de l'encoche, où l'ergot est introduit dans l'encoche correspondante en alignant les faces planes de l'ergot avec les faces planes de l'encoche pour guider l'insertion de l'ergot puis l'ergot est apte à pivoter dans l'encoche par contact au moins partiel entre les faces arquées de l'ergot et le fond arqué de l'encoche, l'entrée rétrécie empêchant une sortie de l'ergot hors de l'encoche.

Ainsi, grâce à ce caractère démontable de la poignée et du système d'actionnement, il est possible de remplacer au moins en partie le système d'actionnement, puis de le remettre en place sur la poignée et sur le manche, afin de pouvoir s'adapter à un autre type de cupule.

Grâce à ces ergots et encoches, il est aisé de démonter la poignée et de faire venir avec elle le système d'actionnement, pour l'adaptation au type de cupule.

En outre, du fait des conformations spécifiques des ergots et des encoches, chaque ergot est bloqué et pivotant dans l'encoche correspondante tant que les faces planes de l'ergot se sont pas alignées avec les faces planes de l'encoche, garantissant un non démontage lors de l'utilisation normale de l'ancillaire. Il est à noter que l'ergot est introduit dans l'encoche correspondante plus précisément en alignant un plan de symétrie des deux faces planes opposées de l'ergot avec un plan de symétrie des deux faces planes en regard de l'encoche.

Selon une possibilité de l'invention, la partie échancrée de la bordure inférieure, entre les deux parois en vis-à-vis, traverse le manche jusqu'à la bordure supérieure, afin de permettre à la biellette incurvée de traverser le manche, en étant insérée du côté de la bordure inférieure, jusqu'à ce que les ergots de la poignée soient réceptionnés à l'intérieur des encoches prévus du côté de la bordure inférieure.

Selon une autre possibilité de l'invention, la bordure supérieure présente une fente allongée s'étendant, le long de la partie proximale incurvée, à partir de la partie échancrée débouchant dans la bordure supérieure jusqu'à la tête de préhension, où la biellette incurvée est déplaçable au moins partiellement à l'intérieur de ladite fente allongée, avec un guidage en translation de la biellette incurvée à l'intérieur de la tête de préhension qu'elle traverse.

Dans une réalisation avantageuse, le dispositif de transmission mécanique comprend en outre une biellette intermédiaire présentant deux extrémités opposées comprenant une extrémité proximale articulée sur la biellette incurvée selon un premier axe de pivotement et une extrémité distale articulée sur la poignée selon un second axe de pivotement, où la biellette intermédiaire présente un axe longitudinal qui relie le premier axe de pivotement au second axe de pivotement, et en ce que la position déployée et la position rabattue sont des positions stables aux extrémités d'une course totale de basculement de la poignée dans laquelle est incluse une position intermédiaire instable de la poignée correspondant à un point dur de rebroussement vers la position déployée ou vers la position rabattue, ladite position intermédiaire étant marquée par le passage de l'axe longitudinal de la biellette intermédiaire sur l'axe de pivotement de la poignée.

Ainsi, grâce à cet ancillaire, le chirurgien ressent un point dur entre la position déployée et la position rabattue de la poignée, où :
- lorsque la poignée est déplacée de la position déployée vers la position rabattue, tant que le chirurgien n'a pas passé le point dur, la poignée tend naturellement à revenir vers la position déployée sous l'effet du dispositif de transmission mécanique et de la coopération entre l'organe de préhension et la tête de préhension et, une fois qu'il a passé le point dur, la poignée tend naturellement à aller vers la position rabattue à nouveau sous l'effet du dispositif de transmission mécanique et de la coopération entre l'organe de préhension et la tête de préhension ; et
- lorsque la poignée est déplacée de la position rabattue vers la position déployée, tant que le chirurgien n'a pas passé le point dur, la poignée tend naturellement à revenir vers la position rabattue sous l'effet du dispositif de transmission mécanique et de la coopération entre l'organe de préhension et la tête de préhension et, une fois qu'il a passé le point dur, la poignée tend naturellement à aller vers la position déployée à nouveau sous l'effet du dispositif de transmission mécanique et de la coopération entre l'organe de préhension et la tête de préhension.

De cette manière, le chirurgien a un ressenti sur le niveau de coopération entre l'organe de préhension et la tête de préhension, ainsi sur le stade de verrouillage/déverrouillage de la cupule sur l'organe de préhension, ce qui lui est d'une grande aide pour maîtriser au mieux son geste chirurgical, en particulier lors d'une pose quasiment à l'aveugle.

Il est à noter que ce dispositif de transmission mécanique avec la biellette intermédiaire, et donc avec le point dur, peut être envisagée indépendamment du caractère démontable ou amovible de la poignée, et peut ainsi être envisagée avec une poignée montée pivotante et non amovible sur le manche.

Selon une caractéristique, la poignée présente, à une première extrémité articulée sur le manche, deux platines en vis-à-vis encadrant une fente à l'intérieur de laquelle est articulée l'extrémité distale de la biellette intermédiaire selon un second axe de pivotement.

Selon une autre caractéristique, les ergots font saillie extérieurement des deux platines respectives.

L'invention concerne également l'une au moins des caractéristiques suivantes :
- en passant de la position déployée vers la position rabattue, la poignée est pivotée en direction de la surface d'impaction ; et
- dans la position intermédiaire de la poignée, l'extrémité proximale et l'extrémité distale de la biellette intermédiaire sont disposées de part et d'autre de l'axe de pivotement de la poignée.

Dans une réalisation particulière, la tête de préhension supporte une platine conique, et l'organe de préhension comprend un palet expansible présentant une surface d'appui propre à coopérer avec la platine conique, de sorte que :
- dans la position déployée de la poignée, la surface d'appui du palet expansible est éloignée de la platine conique afin que le palet expansible occupe une configuration de repos non expansé pour permettre un déverrouillage de la cupule sur le palet expansible ; et
- dans la position rabattue de la poignée, la surface d'appui du palet expansible est pressée contre la platine conique afin que le palet expansible occupe une configuration expansée pour permettre un verrouillage de la cupule sur le palet expansible par friction.

Selon une possibilité, le palet expansible se présente sous la forme d'un dôme substantiellement hémisphérique en matériau élastiquement déformable et présentant des rainures en longitude.

L'invention se rapporte aussi à un kit de pose de plusieurs types de cupule pour implant cotyloïdien de prothèse totale de hanche, ledit kit comprenant un ancillaire conforme à l'invention, et comprenant au moins un autre organe de préhension associé à un autre système d'actionnement, cet autre système d'actionnement étant adapté pour être monté de manière amovible sur le manche de l'ancillaire en lieu et place du système d'actionnement de l'ancillaire.

Ainsi, avec un tel kit, il est possible d'assurer la pose d'au moins deux types de cupule, avec un manche commun et au moins un premier ensemble organe de préhension/système d'actionnement adapté pour un premier type de cupule, et un deuxième ensemble organe de préhension/système d'actionnement adapté pour un deuxième type de cupule, et où le premier ensemble est monté sur le manche pour une utilisation avec le premier type de cupule, et ce premier ensemble est démonté et remplacé par le deuxième ensemble qui est alors monté sur le manche pour une utilisation avec le deuxième type de cupule.

D'autres caractéristiques et avantages de la présente invention apparaîtront à la lecture de la description détaillée ci-après, d'un exemple de mise en oeuvre non limitatif, faite en référence aux figures annexées dans lesquelles :
- la figure 1 est une vue schématique de côté d'un ancillaire conforme à l'invention, avec une tige de positionnement en place et avec la poignée en position rabattue ;
- la figure 2 est une vue schématique de côté de l'ancillaire de la figure 1, avec la poignée en position rabattue et avec l'organe de préhension en place ;
- les figures 3 à 5 sont des vues schématiques respectivement de côté (figure 3), de dessous (figure 4) et de dessus (figure 5) du manche seul de l'ancillaire des figures 1 et 2 ;
- les figures 6 à 8 sont des vues schématiques respectivement de côté (figure 6), en perspective de dessous (figure 7) et de dessous (figure 8) du système d'actionnement seul de l'ancillaire des figures 1 et 2 ;
- la figure 9 est une vue schématique en perspective de dessous de la poignée seule du système d'actionnement de la figure 6 ;
- la figure 10 est une vue schématique de côté de la platine conique et de l'organe de préhension de type palet expansible pour l'ancillaire de la figure 1 ;
- les figures 11 à 16 sont des vues schématiques de l'ancillaire de la figure 1 lors d'étapes successives de montage de l'ancillaire, avec :

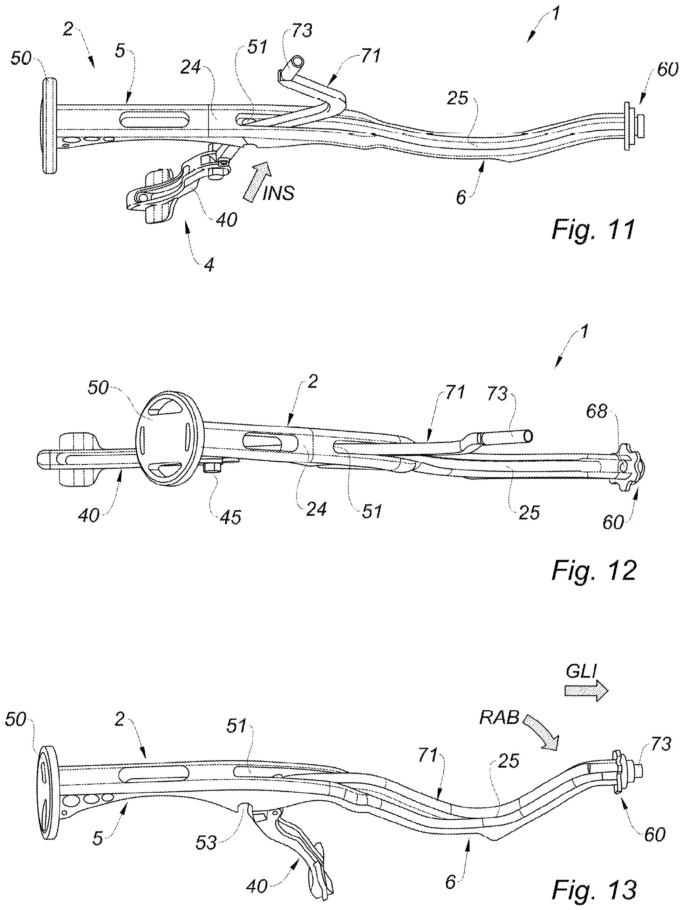

   - les figures 11 et 12 illustrent une première étape de montage au cours de laquelle la biellette incurvée est insérée à travers la partie échancrée du manche ;

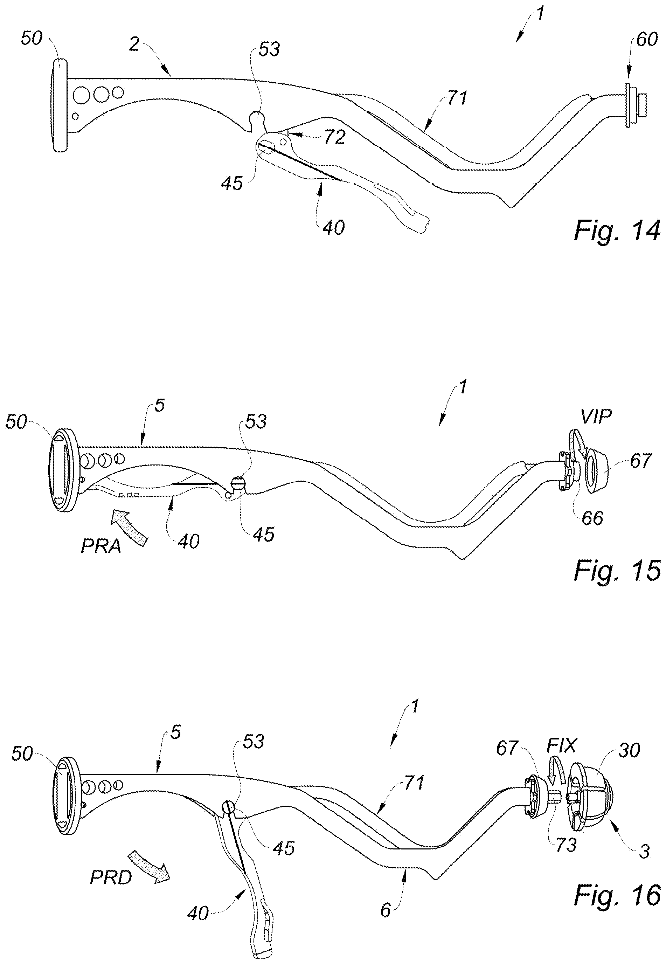

   - les figures 13 et 14 illustrent une deuxième étape de montage au cours de laquelle la biellette incurvée est engagée dans la fente allongée du manche et à travers la tête de préhension du manche, avec un pré-positionnement des ergots de la poignée en face des encoches concernées du manche ;
   - la figure 15 illustre une troisième étape de montage au cours de laquelle les ergots de la poignée sont engagés dans les encoches du manche, et la poignée est actionnée vers la position rabattue pour pouvoir fixer la platine conique sur la tête de préhension ;
   - la figure 16 illustre une quatrième étape de montage au cours de laquelle la poignée est actionnée vers la position déployée afin de pouvoir fixer l'organe de préhension sur l'extrémité proximale de la biellette incurvée ;

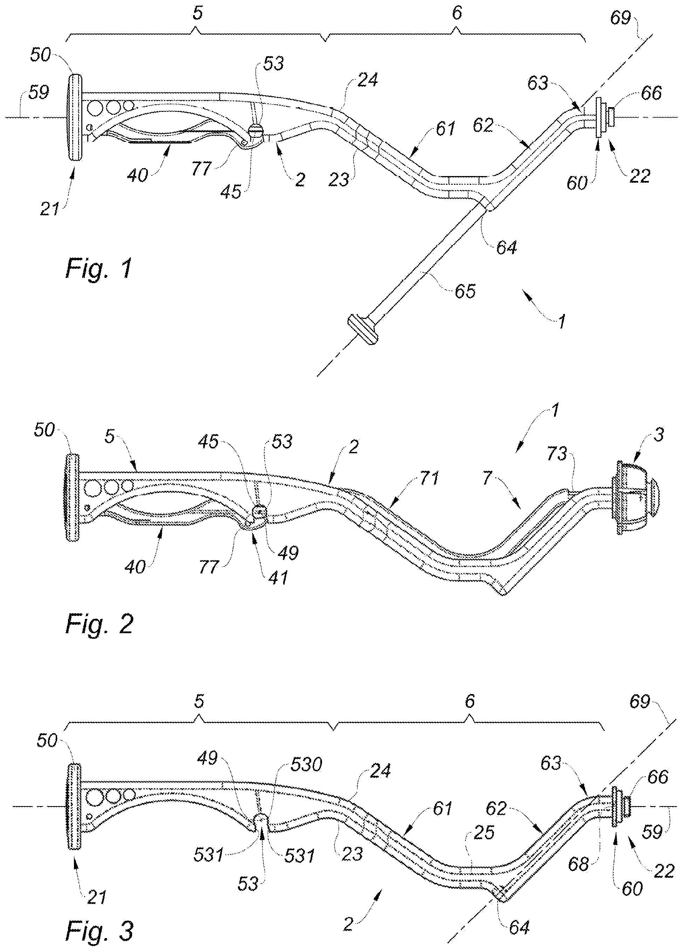

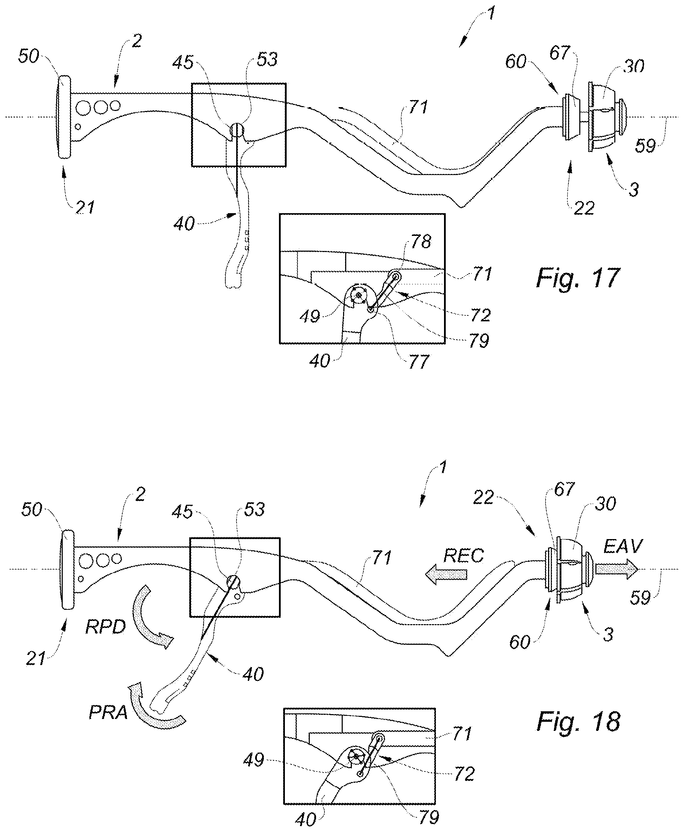

- les figures 17 à 21 sont des vues schématiques en perspective de l'ancillaire de la figure 1 lors de positions successives de la poignée en partant de la position déployée (associée à un déverrouillage d'une cupule) jusqu'à la position rabattue (associée à un verrouillage de la cupule), la cupule n'étant pas illustrée et chaque figure comprenant un zoom en transparence sur la biellette intermédiaire, avec :
   - la figure 17 illustre la position déployée, qui fait directement suite à la quatrième étape de montage illustrée sur la figure 16, avec l'organe de préhension (ou palet expansible) qui occupe une configuration de repos non expansé ;
   - la figure 18 illustre une position entre la position déployée et la position intermédiaire instable, après que la poignée ait commencé à être rabattue et que donc l'organe de préhension ait commencé à s'expanser par coopération avec le palet conique ;

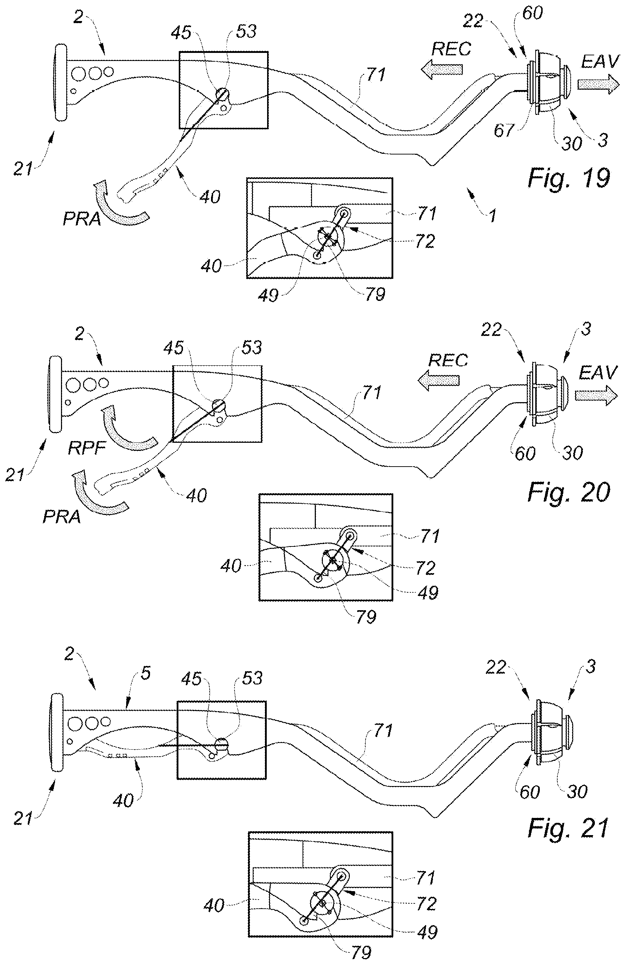

   - la figure 19 illustre la position intermédiaire instable de la poignée correspondant à un point dur de rebroussement vers la position déployée ou vers la position rabattue ;
   - la figure 20 illustre une position entre la position intermédiaire instable et la position rabattue, après que la poignée ait continué à être rabattue passé le point dur ; et
   - la figure 21 illustre la position rabattue, avec l'organe de préhension qui occupe une configuration expansée.

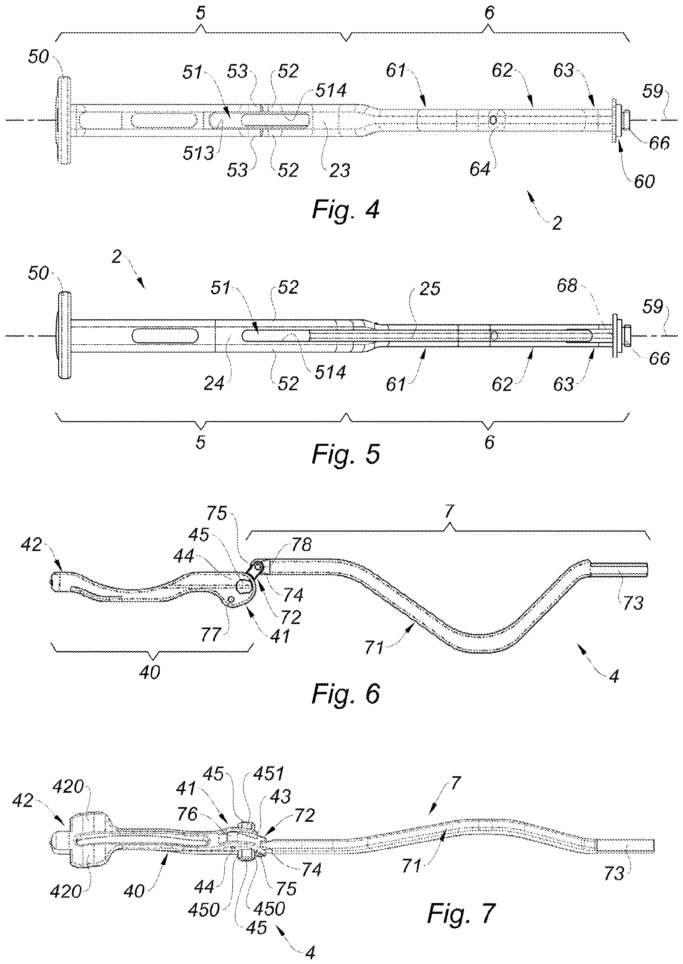

En référence aux figures 1 à 10, un ancillaire 1 conforme à l'invention comprend un manche 2 de manipulation, un organe de préhension 3 et un système d'actionnement 4.

Le manche 2 est réalisé d'un seul tenant dans un matériau métallique et comprend une partie distale 5 conçue pour une saisie manuelle et présentant une surface d'impaction 50 disposée à une extrémité distale 21 libre du manche 2. Cette partie distale 5 s'étend selon une direction principale 59.

Le manche 2 comprend en outre une partie proximale 6 qui prolonge la partie distale 5 jusqu'à une extrémité proximale 22 libre du manche 1. La partie proximale 6 est une partie incurvée se terminant par une tête de préhension 60.

Cette partie proximale 6 comprend successivement, en partant de la partie distale 5 :
- un premier tronçon 61 courbé ;
- un deuxième tronçon 62 s'étendant selon une direction secondaire 69 formant un angle avec la direction principale 59 ; et
- un troisième tronçon 63 s'étendant selon une direction parallèle à la direction principale 59 et se terminant par la tête de préhension 60.

Le deuxième tronçon 62 présente, à la jonction avec le premier tronçon 61, une surface de frappe secondaire 64 orthogonale à la direction secondaire 69 pour pouvoir, si nécessaire, appliquer un impact selon cette direction secondaire 69.

Dans la surface de frappe secondaire 64 est prévu un taraudage pour pouvoir, si nécessaire, visser une tige de positionnement 65 (visible sur la seule figure 1) alignée sur la direction secondaire 69 et prévue pour un contrôle de l'orientation de la tête de préhension 60 et donc de la cupule.

Le manche 2 présente une bordure inférieure 23 et une bordure supérieure 24. La surface de frappe secondaire 64 est prévue sur la bordure inférieure 23.

La partie distale 5 du manche 2 présente une partie échancrée 51 dans la bordure inférieure 23 et qui traverse la partie distale 5 de la bordure inférieure 23 jusqu'à la bordure supérieure 24. Cette partie échancrée 51 forme ainsi une fenêtre traversant la partie distale 5 de la bordure inférieure 23 jusqu'à la bordure supérieure 24. Cette partie échancrée 51 présente ainsi une largeur réduite comparativement à la largeur de la bordure inférieure 23 et de la bordure supérieure 24.

Cette partie échancrée 51 débouche sur la bordure inférieure 23 sous la forme d'une ouverture inférieure 513 et débouche sur la bordure supérieure 24 sous la forme d'une ouverture supérieure 514 ; cette ouverture inférieure 513 et cette ouverture supérieure 514 étant communicante.

Cette partie échancrée 51 est délimitée par deux parois 52 en vis-à-vis, où chaque paroi 52 s'étend latéralement de la bordure inférieure 23 et de la bordure supérieure 24.

Du côté de la bordure inférieure 23, les deux parois 51 présentent des encoches 53 en regard l'une de l'autre, de sorte que la partie distale 5 du manche 2 présente deux encoches 53 en vis-à-vis et de part et d'autre de la partie échancrée 51, et en particulier de part et d'autre de l'ouverture inférieure 513.

En référence à la figure 3, chaque encoche 53 est délimitée par une paroi périphérique interne qui présente un fond arqué 530 sur un secteur angulaire supérieur à 180°, et deux faces planes 531 en regard formant une entrée rétrécie à l'intérieur de l'encoche 53.

La bordure supérieure 24 du manche 2 présente une fente allongée 25 qui s'étend à partir de la partie échancrée 51 jusqu'à la tête de préhension 60. Cette fente allongée 25 prolonge ainsi la partie échancrée 51 du côté de la bordure supérieure 24, et prolonge donc l'ouverture supérieure 514. Cette fente allongée 25 présente ainsi une largeur réduite comparativement à la largeur de la bordure supérieure 24, et présente notamment la même largeur que la partie échancrée 51. Cette fente allongée 25 est non traversante, ce qui signifie qu'elle ne traverse pas le manche 2 et ne débouche donc pas dans la bordure inférieure 23.

Cette fente allongée 25 s'étend ainsi tout le long de la partie proximale 6 incurvée, jusqu'à un trou 68 traversant la tête de préhension 60.

Le système d'actionnement 4 comprend une poignée 40 mobile montée pivotante selon un axe de pivotement 49 sur le manche 2. Cet axe de pivotement 49 coïncide avec les centres des fonds arqués 530 des deux encoches 53.

La poignée 40 présente une première extrémité 41 articulée sur le manche 2 et une seconde extrémité 42 sur laquelle agit un utilisateur pour actionner/déplacer la poignée 40.

La première extrémité 41 de la poignée 40 est une extrémité fendue dans la mesure où cette première extrémité 41 présente une fente 43 encadrée par deux platines 44 en vis-à-vis.

Cette première extrémité 41 présente deux ergots 45 saillants extérieurement, et plus précisément en saillie extérieurement des deux platines 44 respectives. Chaque ergot 45 présente une paroi périphérique cylindrique tronquée sur deux côtés opposés pour définir deux faces arquées 450 opposées et deux faces planes 451 opposées.

Ainsi, la poignée 40 peut être montée pivotante et de manière amovible sur le manche 2, avec les ergots 45 qui sont réceptionnés dans les encoches 53 respectives.

Chaque ergot 45 peut pivoter à l'intérieur de l'encoche 53 concernée par contact entre les faces arquées 450 de l'ergot 45 et le fond arqué 530 de l'encoche 53. Lors du pivotement, les entrées rétrécies des encoches 53 empêchent les ergots 45 de sortir hors des encoches et donc empêchent un démontage inopiné de la poignée 40.

En outre, chaque ergot 45 peut être introduit dans l'encoche 53 concerné, lors du montage de la poignée 40, en alignant les faces planes 451 de l'ergot 45 avec les faces planes 531 de l'encoche pour guider l'insertion de l'ergot 45 à l'intérieur de l'encoche 53, autrement dit en alignant un plan de symétrie des deux faces planes 451 opposées de l'ergot 45 avec un plan de symétrie des deux faces planes 531 en regard de l'encoche 53. A l'inverse, chaque ergot 45 peut être retiré hors de l'encoche 53 concerné, lors du démontage de la poignée 40, en alignant les faces planes 451 de l'ergot avec les faces planes 531 de l'encoche pour guider la sortie de l'ergot 45 à l'intérieur de l'encoche 53.

La seconde extrémité 42 de la poignée 40 présente des ailes 420 pour faciliter la préhension.

Le système d'actionnement 4 comprend en outre un dispositif de transmission 7 mécanique reliant la poignée 40 à l'organe de préhension 3.

Ce dispositif de transmission 7 comprend une biellette incurvée 71 et une biellette intermédiaire 72 articulées entre elles.

En situation, la biellette incurvée 71 est guidée en déplacement dans la fente allongée 25 ménagée en partie sur la partie proximale 6 du manche 2 et présente une extrémité proximale 73 traversant la tête de préhension 60 pour coopérer avec l'organe de préhension 3. A ce titre, l'extrémité proximale 73 traverse le trou 68 traversant la tête de préhension 60 dans le prolongement de la fente allongée 25. Lors des manipulations/actionnements de la poignée 40, la biellette incurvée 71 est guidée en translation à l'intérieur du trou 68 traversant ménagé dans la tête de préhension 60. Autrement dit, l'extrémité proximale 73 est montée coulissante à l'intérieur de ce trou 68 selon une direction de coulissement parallèle à la direction principale 59.

L'organe de préhension 3 est fixé de manière amovible sur l'extrémité proximale 73 de la biellette incurvée 71, afin de permettre le démontage de la poignée 40 et du système d'actionnement 4 hors du manche 2.

La biellette incurvée 71 épouse sensiblement la forme de la fente allongée 25 et donc de la partie proximale 6 du manche 2. La biellette incurvée 71 est reçue au moins partiellement à l'intérieur de cette fente allongée 25. La biellette incurvée 71 présente une extrémité distale 74 articulée sur la biellette intermédiaire 72.

La biellette intermédiaire 72 présente deux extrémités opposées comprenant une extrémité proximale 75 articulée sur l'extrémité distale 74 de la biellette incurvée 71 selon un premier axe de pivotement 78, et une extrémité distale 76 articulée sur la poignée 40 selon un second axe de pivotement 77. L'extrémité proximale 75 et l'extrémité distale 76 de la biellette intermédiaire 72 sont reliées par un axe longitudinal 79 qui intersecte le premier axe de pivotement 78 et le second axe de pivotement 77.

A ce titre, l'extrémité distale 76 de la biellette intermédiaire 72 est articulée à l'intérieur de la fente 43 ménagée sur la première extrémité 41 de la poignée 40. Ainsi, la biellette intermédiaire 72 s'étend en partie à l'intérieur de cette fente 43, entre les deux platines 44 en vis-à-vis.

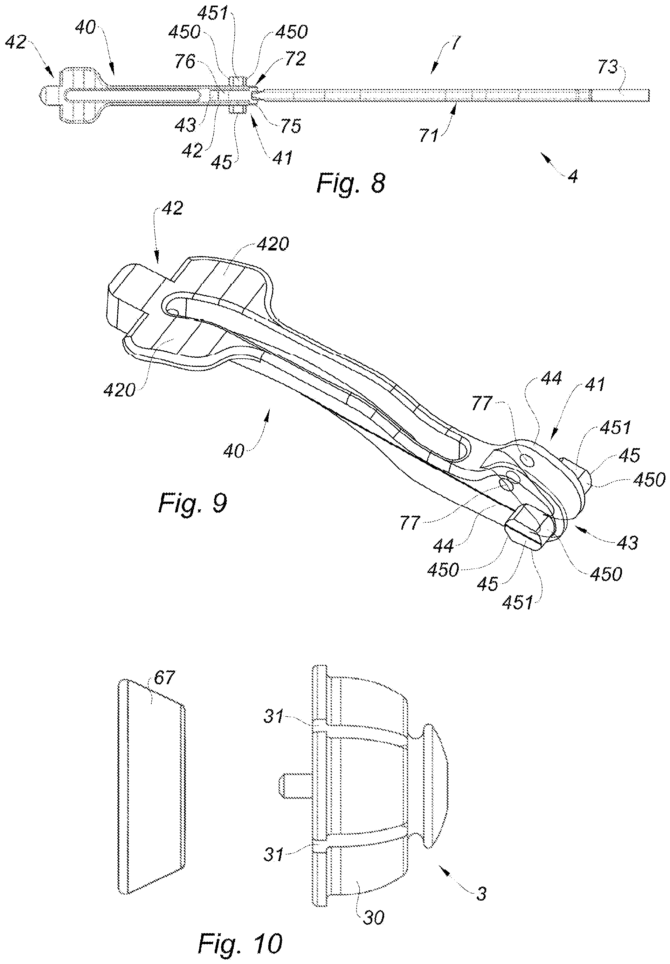

Comme visible sur la figure 9, le second axe de pivotement 77 est matérialisé par deux orifices en vis-à-vis, prévus sur les platines 44 et propres à supporter un axe matériel sur lequel est articulée l'extrémité distale 76 de la biellette intermédiaire 72.

La tête de préhension 60 présente un manchon fileté 66 sur lequel est vissée une platine conique 67 dont le diamètre externe est décroissant en s'éloignant de l'extrémité proximale 22 du manche 2.

L'organe de préhension 3 comprend un palet expansible 30 présentant une surface d'appui propre à coopérer avec la platine conique 67. Ce palet expansible 30 se présente sous la forme d'un dôme substantiellement hémisphérique en matériau élastiquement déformable et présentant des rainures 31 en longitude.

Ce palet expansible 30 est propre à coopérer avec la tête de préhension 60, et plus précisément avec la platine conique 67, pour la préhension d'une cupule (non illustrée) pour implant cotyloïdien de prothèse totale de hanche.

La suite de la description porte sur le montage de l'ancillaire 1, en référence aux figures 11 à 16.

Dans une première étape de montage illustrée sur les figures 11 et 12, la biellette incurvée 71 du système d'actionnement 4 est insérée à travers la partie échancrée 51 du manche 2, comme schématisé par la flèche INS, avec une insertion qui se fait du côté de la bordure inférieure 23 du manche 2. Autrement dit, la biellette incurvée 71 est engagée à travers l'ouverture inférieure 513 et débouche ensuite à travers l'ouverture supérieure 514.

Dans une deuxième étape de montage illustrée sur les figures 13 et 14, la biellette incurvée 71 est rabattue dans la fente allongée 25 du manche 2, comme schématisé par la flèche RAB, puis est glissée dans la fente allongée 25, comme schématisé par la flèche GLI, de sorte que son extrémité proximale 73 traverse la tête de préhension 60. A l'issue de cette deuxième étape de montage, les ergots 45 de la poignée 40 sont pré-positionnés en face des encoches 53 concernées du manche 2.

Dans une troisième étape de montage illustrée sur la figure 15, les ergots 45 de la poignée 40 sont engagés dans les encoches 53 du manche 2, et la poignée 40 est actionnée vers une position rabattue en étant pivotée selon un sens de pivotement appelé par la suite sens de fermeture (sens horaire sur les figures), en direction de la surface d'impaction 50, comme schématisé par la flèche PRA. A l'issue de ce pivotement de la poignée 40, la platine conique 67 est vissée sur le manchon fileté 66, comme schématisé par la flèche VIP, fixant ainsi la platine conique 67 sur la tête de préhension 60.

Dans une quatrième étape de montage illustrée sur la figure 16, la poignée 40 est actionnée vers une position déployée en étant pivotée selon un sens de pivotement appelé par la suite sens d'ouverture (sens anti-horaire sur les figures), opposé au sens de fermeture, comme schématisé par la flèche PRD, ce qui conduit à ce que l'extrémité proximale 73 de la biellette incurvée 71 dépasse hors de la platine conique 67, permettant ainsi de fixer le palet expansible 30 sur cette extrémité proximale 73, comme schématisé par la flèche FIX.

La suite de la description porte sur l'utilisation de l'ancillaire 1, en référence aux figures 17 à 21.

La poignée 40 est actionnable en pivotement, dans les encoches 53, par l'utilisateur entre :
- la position déployée (visible sur la figure 17) dans laquelle la poignée 40 s'étend sensiblement perpendiculairement à la direction principale 59, de manière à ce que l'organe de préhension 3 soit éloigné de la tête de préhension 60 de sorte que la surface d'appui du palet expansible 30 est éloignée de la platine conique 67 afin que le palet expansible 30 occupe une configuration de repos non expansé pour permettre un déverrouillage de la cupule sur le palet expansible 60 ; et
- la position rabattue (visible sur la figure 21) dans laquelle la poignée 40 s'étend sensiblement parallèlement à la direction principale 59 en étant rabattue contre la partie distale 6, de manière à ce que l'organe de préhension 3 coopère avec la tête de préhension 60 de sorte que la surface d'appui du palet expansible 30 est pressée contre la platine conique 67 afin que le palet expansible 30 occupe une configuration expansée pour permettre un verrouillage de la cupule sur le palet expansible 60 par friction.

En référence à la figure 17, lors du montage de la cupule sur l'ancillaire 1, la poigné 40 est initialement dans la position déployée, autorisant l'insertion du palet expansible 30 dans la cupule. Dans cette position déployée, comme visible sur le zoom de la figure 17, l'axe longitudinal 79 de la biellette intermédiaire 72 se situe sur un côté proximal vis-à-vis de l'axe de pivotement 49.

En référence à la figure 18, pour verrouiller la cupule sur le palet expansible 30, la poignée 40 est actionnée pour être rabattue contre la partie distale 5 du manche 2, en étant pivotée selon le sens de fermeture, comme schématisé par la flèche PRA. Ce pivotement de la poignée 40 entraîne alors la translation de la biellette incurvée 71 en direction de l'extrémité distale 21 du manche 2, autrement dit entraîne un recul de la biellette incurvée 71, comme schématisé par la flèche REC. Ainsi, le palet expansible 30 vient au contact de la platine conique 67 et est pressé contre cette platine conique 67, contribuant à l'expansion du palet expansible 30 et donc au verrouillage de la cupule. Le palet expansible 30, pressé contre la platine conique 67, imprime alors un effort (schématisé par la flèche EAV) qui tend à ramener la biellette incurvée dans la configuration de la figure 17, autrement dit qui tend à faire translater la biellette incurvée 71 en direction de l'extrémité proximale 22 du manche 2, c'est-à-dire à faire avancer la biellette incurvée 71. Cet effort EAV se traduit par un effort qui tend à faire pivoter la poignée 40 selon le sens d'ouverture, comme schématisé par la flèche RPD, pour un retour de la poignée 40 dans la position déployée. Au stade de pivotement illustré sur la figure 18, si l'utilisateur relâche son effort sur la poignée 40, celle-ci tendra à revenir naturellement vers la position déployée du fait de l'effort EAV imprimé par le palet expansible 30. Dans cette position de début de pivotement, comme visible sur le zoom de la figure 18, l'axe longitudinal 79 de la biellette intermédiaire 72 se situe encore sur le côté proximal vis-à-vis de l'axe de pivotement 49.

En référence à la figure 19, la poignée 40 continue d'être actionnée par pivotement selon le sens de fermeture, comme schématisé par la flèche PRA, de sorte que la biellette incurvée 71 subit un recul, comme schématisé par la flèche REC. Le palet expansible 30 continue ainsi d'être pressé contre la platine conique 67, et imprime alors l'effort EAV qui tend à faire avancer la biellette incurvée 71. Sur la figure 19, et comme visible en particulier sur son zoom, la poignée 40, dans sa course totale de basculement de la position déployée de la figure 17 vers la position rabattue de la figure 21, a atteint une position intermédiaire instable qui correspond à un point dur de rebroussement vers la position déployée ou vers la position rabattue, cette position intermédiaire étant marquée par le passage de l'axe longitudinal 79 de la biellette intermédiaire 72 sur l'axe de pivotement 49 de la poignée 40. Autrement dit, dans cette position intermédiaire instable, l'axe longitudinal 79 intersecte l'axe de pivotement 49. Cette position intermédiaire instable correspond à une position d'équilibre instable où la poignée est à l'équilibre malgré l'effort EAV. Ainsi, si l'utilisateur relâche son effort sur la poignée 40, celle-ci reste dans la position intermédiaire instable. Par contre, cette position d'équilibre est instable dans la mesure où :
- comme expliqué précédemment en référence à la figure 18, avant cette position d'équilibre instable, si l'utilisateur relâche son effort sur la poignée 40, celle-ci tendra à revenir naturellement vers la position déployée du fait de l'effort EAV ; et
- comme expliqué ultérieurement en référence à la figure 20, après cette position d'équilibre instable, si l'utilisateur relâche son effort sur la poignée 40, celle-ci tendra à revenir naturellement vers la position rabattue du fait de l'effort EAV.

Ainsi, cette position intermédiaire instable correspond à un point dur de rebroussement :
- vers la position déployée, si un actionnement, même minime, est exercé sur la poignée 40 pour la faire pivoter selon le sens de fermeture ;
- vers la position rabattue, si un actionnement, même minime, est exercé sur la poignée 40 pour la faire pivoter selon le sens d'ouverture.

En référence à la figure 20, la poignée 40 continue d'être actionnée par pivotement selon le sens de fermeture, comme schématisé par la flèche PRA, de sorte que la biellette incurvée 71 subit un recul, comme schématisé par la flèche REC. Le palet expansible 30 continue ainsi d'être pressé contre la platine conique 67, et imprime alors toujours l'effort EAV qui tend à faire avancer la biellette incurvée 71. Cependant, à la différence de celle de la figure 18, la poignée se trouve cette fois après la position intermédiaire instable où, comme visible sur le zoom de la figure 20, l'axe longitudinal 79 de la biellette intermédiaire 72 se situe cette fois sur le côté distal vis-à-vis de l'axe de pivotement 49 (autrement dit sur le côté opposé par rapport au côté proximal évoqué pour la figure 18). Ainsi, ayant passé le poigné dur, cet effort EAV se traduit par un effort qui tend à faire pivoter la poignée 40 selon le sens de fermeture, comme schématisé par la flèche RPF, pour un envoi de la poignée 40 vers la position rabattue. Au stade de pivotement illustré sur la figure 20, si l'utilisateur relâche son effort sur la poignée 40, celle-ci tendra à aller naturellement vers la position rabattue du fait de l'effort EAV imprimé par le palet expansible 30.

En référence à la figure 21, à la fin du montage de la cupule sur l'ancillaire 1, la poigné 40 est dans la position rabattue, verrouillant ainsi le palet expansible 30 à l'intérieur de la cupule. Dans cette position rabattue, comme visible sur le zoom de la figure 21, l'axe longitudinal 79 de la biellette intermédiaire 72 se situe encore sur le côté distal vis-à-vis de l'axe de pivotement 49. Ainsi, tout effort exercé sur la poignée 40 pour la ramener vers la position déployée se verra opposé un effort qui tendra à la ramener, à l'opposé, vers la position rabattue, sécurisant ainsi la position rabattue durant les manipulations, sans risque d'ouverture inopinée.

## Revendications

1. Ancillaire (1) de pose d'une cupule pour implant cotyloïdien de prothèse totale de hanche, du type comportant :
- un manche (2) de manipulation comprenant, d'une part, une partie distale (5) conçue pour une saisie manuelle et présentant une surface d'impaction (50) et, d'autre part, une partie proximale (6) incurvée se terminant par une tête de préhension (60) ;
- un organe de préhension (3) propre à coopérer avec la tête de préhension (60) pour la préhension de la cupule ; et
- un système d'actionnement (4) comprenant une poignée (40) mobile montée pivotante selon un axe de pivotement (49) sur le manche (2) et un dispositif de transmission mécanique (7) reliant ladite poignée (40) audit organe de préhension (3), ledit dispositif de transmission mécanique (7) comprenant une biellette incurvée (71) guidée en déplacement par la partie proximale (6) du manche (2) et traversant la tête de préhension (60) pour coopérer avec ledit organe de préhension (3) ;
où, pour déplacer l'organe de préhension (3), la poignée (40) est actionnable entre :
- une position déployée de manière à ce que l'organe de préhension (3) soit éloigné de la tête de préhension (60) pour permettre un déverrouillage de la cupule sur l'organe de préhension (3) ; et
- une position rabattue de manière à ce que l'organe de préhension (3) coopère avec la tête de préhension (60) pour permettre un verrouillage de la cupule sur l'organe de préhension (3) ;
dans lequel la poignée (40) est montée pivotante et de manière amovible sur le manche (2) et l'organe de préhension (3) est fixé de manière amovible sur la biellette incurvée (71), afin de permettre le démontage de la poignée (40) et du système d'actionnement (4) hors du manche (2),
dans lequel le manche (2) présente une bordure inférieure (23) et une bordure supérieure (24), et la poignée (40) est articulée sur une partie échancrée (51) de ladite bordure inférieure (23), ladite partie échancrée (51) étant délimitée par deux parois (52) en vis-à-vis, où les deux parois (52) présentent des encoches (53) en regard l'une de l'autre, et la poignée (40) présente, à une première extrémité (41) articulée sur le manche (2), deux ergots (45) saillants réceptionnés dans les encoches (53) respectives, de sorte que, d'une part, lesdits ergots (45) puissent pivoter à l'intérieur desdites encoches (53) et, d'autre part, lesdits ergots (45) puissent être retirés hors desdites encoches (53) lors du démontage de la poignée (40) et du système d'actionnement (4) hors du manche (2) ;
ledit ancillaire (1) étant **caractérisé en ce que** chaque ergot (45) présente une paroi périphérique cylindrique tronquée sur deux côtés opposés pour définir deux faces planes (451) opposées et deux faces arquées (450) opposées, et chaque encoche (53) est délimitée par une paroi périphérique interne qui présente un fond arqué (530) et deux faces planes (531) en regard formant une entrée rétrécie à l'intérieur de l'encoche (53), où l'ergot (45) est introduit dans l'encoche (53) correspondante en alignant les faces planes (451) de l'ergot (45) avec les faces planes (531) de l'encoche (53) pour guider l'insertion de l'ergot (45) puis l'ergot (45) est apte à pivoter dans l'encoche (53) par contact au moins partiel entre les faces arquées (450) de l'ergot (45) et le fond arqué (530) de l'encoche (53), l'entrée rétrécie empêchant une sortie de l'ergot (45) hors de l'encoche (53).

2. Ancillaire (1) selon la revendication 1, dans lequel la partie échancrée (51) de la bordure inférieure (23), entre les deux parois (52) en vis-à-vis, traverse le manche (2) jusqu'à la bordure supérieure (24), afin de permettre à la biellette incurvée (71) de traverser le manche (2), en étant insérée du côté de la bordure inférieure (23), jusqu'à ce que les ergots (45) de la poignée (40) soient réceptionnés à l'intérieur des encoches (53) prévus du côté de la bordure inférieure (23).

3. Ancillaire (1) selon la revendication 2, dans lequel la bordure supérieure (24) présente une fente allongée (25) s'étendant, le long de la partie proximale (6) incurvée, à partir de la partie échancrée (51) débouchant dans la bordure supérieure (24) jusqu'à la tête de préhension (60), où la biellette incurvée (71) est déplaçable au moins partiellement à l'intérieur de ladite fente allongée (25), avec un guidage en translation de la biellette incurvée (71) à l'intérieur de la tête de préhension (60) qu'elle traverse.

4. Ancillaire (1) selon l'une quelconque des revendications précédentes, dans lequel le dispositif de transmission mécanique (7) comprend en outre une biellette intermédiaire (72) présentant deux extrémités opposées comprenant une extrémité proximale (75) articulée sur la biellette incurvée (71) selon un premier axe de pivotement (78) et une extrémité distale (76) articulée sur la poignée (40) selon un second axe de pivotement (77), où la biellette intermédiaire (72) présente un axe longitudinal (79) qui relie le premier axe de pivotement (78) au second axe de pivotement (77), et où la position déployée et la position rabattue sont des positions stables aux extrémités d'une course totale de basculement de la poignée (40) dans laquelle est incluse une position intermédiaire instable de la poignée (40) correspondant à un point dur de rebroussement vers la position déployée ou vers la position rabattue, ladite position intermédiaire étant marquée par le passage de l'axe longitudinal (79) de la biellette intermédiaire (72) sur l'axe de pivotement (49) de la poignée (40)

5. Ancillaire (1) selon la revendication 4, dans lequel la poignée (40) présente, à une première extrémité (41) articulée sur le manche (2), deux platines (44) en vis-à-vis encadrant une fente (43) à l'intérieur de laquelle est articulée l'extrémité distale (76) de la biellette intermédiaire (72) selon un second axe de pivotement (77).

6. Ancillaire (1) selon la revendication 5, dans lequel les ergots (45) font saillie extérieurement des deux platines (44) respectives.

7. Ancillaire (1) selon l'une quelconque des revendications précédentes, dans lequel, en passant de la position déployée vers la position rabattue, la poignée (40) est pivotée en direction de la surface d'impaction (50).

8. Ancillaire (1) selon l'une quelconque des revendications précédentes, dans lequel la tête de préhension (60) supporte une platine conique (67), et l'organe de préhension (3) comprend un palet expansible (30) présentant une surface d'appui propre à coopérer avec la platine conique (67), de sorte que :
- dans la position déployée de la poignée (40), la surface d'appui du palet expansible (30) est éloignée de la platine conique (67) afin que le palet expansible (30) occupe une configuration de repos non expansé pour permettre un déverrouillage de la cupule sur le palet expansible (30) ; et
- dans la position rabattue de la poignée (40), la surface d'appui du palet expansible (30) est pressée contre la platine conique (67) afin que le palet expansible (30) occupe une configuration expansée pour permettre un verrouillage de la cupule sur le palet expansible (30) par friction.

9. Ancillaire (1) selon la revendication 8, dans lequel le palet expansible (30) se présente sous la forme d'un dôme substantiellement hémisphérique en matériau élastiquement déformable et présentant des rainures (31) en longitude.

10. Kit de pose de plusieurs types de cupule pour implant cotyloïdien de prothèse totale de hanche, ledit kit comprenant un ancillaire (1) conforme à l'une quelconque des revendications précédentes, et comprenant au moins un autre organe de préhension associé à un autre système d'actionnement, ledit autre système d'actionnement étant adapté pour être monté de manière amovible sur le manche (2) dudit ancillaire (1) en lieu et place du système d'actionnement (4) dudit ancillaire (1).

## Patentansprüche

1. Hilfsvorrichtung (1) zum Implantieren einer Pfanne für ein Hüftgelenkpfannenimplantat einer Totalhüftprothese des Typs, aufweisend:
- einen Bediengriff (2), umfassend zum einen einen distalen Teil (5), der für ein manuelles Ergreifen konstruiert ist und eine Stoßfläche (50) aufweist und zum anderen einen gekrümmten proximalen Teil (6), der mit einem Greifkopf (60) endet;
- ein Greiforgan (3), das imstande ist, mit dem Greifkopf (60) für das Ergreifen der Pfanne zusammenzuwirken; und
- ein Betätigungssystem (4), umfassend ein Handstück (40), das gemäß einer Schwenkachse (49) schwenkend beweglich auf dem Griff (2) angebracht ist und eine mechanische Übertragungsvorrichtung (7), die das Handstück (40) mit dem Greiforgan (3) verbindet, wobei die mechanische Übertragungsvorrichtung (7) eine gekrümmte Stange (71) umfasst, die von dem proximalen Teil (6) des Griffs (2) verlagerungsgeführt wird und den Greifkopf (60) durchquert, um mit dem Greiforgan (3) zusammenzuwirken;
wobei, um das Greiforgan (3) zu verlagern, das Handstück (40) betätigbar ist zwischen:
- einer hochgeklappten Stellung, so dass das Greiforgan (3) vom Greifkopf (60) entfernt ist, um ein Entriegeln der Pfanne auf dem Greiforgan (3) zu erlauben; und
- einer heruntergeklappten Stellung, so dass das Greiforgan (3) mit dem Greifkopf (60) zusammenwirkt, um ein Verriegeln der Pfanne auf dem Greiforgan (3) zu erlauben;
wobei das Handstück (40) schwenkend und lösbar auf dem Griff (2) angebracht ist und das Greiforgan (3) lösbar auf der gekrümmten Stange (71) befestigt ist, um die Demontage des Handstücks (40) und des Betätigungssystems (4) vom Griff (2) zu erlauben,
wobei der Griff (2) einen unteren Rand (23) und einen oberen Rand (24) aufweist und das Handstück (40) auf einem ausgebuchteten Teil (51) des unteren Randes (23) angelenkt ist, wobei der ausgebuchtete Teil (51) von zwei gegenüberliegenden Wänden (52) begrenzt ist, wobei die zwei Wände (52) einander zugewandte Kerben (53) aufweisen und das Handstück (40) an einem ersten, auf dem Griff (2) angelenkten Ende (41) zwei hervorstehende Zapfen (45) aufweist, die in den jeweiligen Kerben (53) aufgenommen sind, so dass zum einen die Zapfen (45) im Inneren der Kerben (53) schwenken können und zum anderen die Zapfen (45) bei der Demontage des Handstücks (40) und des Betätigungssystems (4) vom Griff (2) aus den Kerben (53) gezogen werden können;
wobei die Hilfsvorrichtung (1) **dadurch gekennzeichnet ist, dass** jeder Zapfen (45) eine zylindrische Umfangswand aufweist, die auf zwei gegenüberliegenden Seiten abgeschnitten ist, um zwei gegenüberliegende ebene Flächen (451) und zwei gegenüberliegende gebogene (450) Flächen zu bilden und jede Kerbe (53) von einer inneren Umfangswand begrenzt ist, die einen gebogenen Boden (530) und zwei einander zugewandte ebene Flächen (531) aufweist, die einen verengten Eingang in das Innere der Kerbe (53) bilden, wobei der Zapfen (45) in die entsprechende Kerbe (53) durch Fluchten der ebenen Flächen (451) des Zapfens (45) mit den ebenen Flächen (531) der Kerbe (53) eingesetzt wird, um das Einsetzen des Zapfens (45) zu führen, dann der Zapfen (45) imstande ist, in der Kerbe (53) durch mindestens teilweisen Kontakt zwischen den gebogenen Flächen (450) des Zapfens (45) und dem gebogenen Boden (530) der Kerbe (53) zu schwenken, wobei der verengte Eingang verhindert, dass der Zapfen (45) die Kerbe (53) verlässt.

2. Hilfsvorrichtung (1) nach Anspruch 1, wobei der ausgebuchtete Teil (51) des unteren Randes (23) zwischen den zwei gegenüberliegenden Wänden (52) den Griff (2) bis zum oberen Rand (24) durchquert, um der gekrümmten Stange (71) zu erlauben, den Griff (2) zu durchqueren, wenn sie von der Seite des unteren Randes (23) eingesetzt ist, bis die Zapfen (45) des Handstücks (40) im Inneren der Kerben (53) eingesetzt sind, die auf der Seite des unteren Randes (23) vorgesehen sind.

3. Hilfsvorrichtung (1) nach Anspruch 2, wobei der obere Rand (24) einen länglichen Schlitz (25) aufweist, der sich entlang des gekrümmten proximalen Teils (6) ab dem ausgebuchteten Teil (51), der im oberen Rand (24) ausmündet, bis zum Greifkopf (60) zu erstrecken, wobei die gekrümmte Stange (71) mindestens teilweise im Inneren des länglichen Schlitzes (25) mit einer translatorischen Führung der gekrümmten Stange (71) im Inneren des Greifkopfs (60), den sie durchquert, verlagerbar ist.

4. Hilfsvorrichtung (1) nach einem der vorangehenden Ansprüche, wobei die mechanische Übertragungsvorrichtung (7) ferner eine Verbindungsstange (72) aufweist, die zwei gegenüberliegende Enden aufweist, umfassend ein proximales Ende (75), das auf der gekrümmten Stange (71) gemäß einer ersten Schwenkachse (78) angelenkt ist, und ein distales Ende (76), das auf dem Handstück (40) gemäß einer zweiten Schwenkachse (77) angelenkt ist, wobei die Verbindungsstange (72) eine Längsachse (79) aufweist, die die erste Schwenkachse (78) mit der zweiten Schwenkachse (77) verbindet, und wobei die hochgeklappte Stellung und die heruntergeklappte Stellung stabile Stellungen an den Enden eines Gesamtschwenkwegs des Handstücks (40) sind, in dem eine instabile Übergangsstellung des Handstücks (40) inbegriffen ist, die einer schwergängigen Umkehrstelle in die hochgeklappte Stellung oder in die heruntergeklappte Stellung entspricht, wobei die Übergangsstellung vom Übergang der Längsachse (79) der Verbindungsstange (72) auf der Schwenkachse (49) des Handstücks (40) gekennzeichnet ist.

5. Hilfsvorrichtung (1) nach Anspruch 4, wobei das Handstück (40) an einem ersten, auf dem Griff (2) angelenkten Ende (41) zwei gegenüberliegende Platten (44) aufweist, die einen Schlitz (43) umschließen, in dessen Inneren das distale Ende (76) der Verbindungsstange (72) gemäß einer zweiten Schwenkachse (77) angelenkt ist.

6. Hilfsvorrichtung (1) nach Anspruch 5, wobei die Zapfen (45) außen aus den zwei jeweiligen Platten (44) hervorstehen.

7. Hilfsvorrichtung (1) nach einem der vorangehenden Ansprüche, wobei das Handstück (40) beim Übergang aus der hochgeklappten Stellung in die heruntergeklappte Stellung in Richtung der Stoßfläche (50) geschwenkt wird.

8. Hilfsvorrichtung (1) nach einem der vorangehenden Ansprüche, wobei der Greifkopf (60) eine konische Platte (67) stützt und das Greiforgan (3) eine expandierbare Scheibe (30) umfasst, die eine Stützfläche aufweist, die imstande ist, mit der konischen Platte (67) zusammenzuwirken, so dass:
- in der hochgeklappten Stellung des Handstücks (40) die Stützfläche der expandierbaren Scheibe (30) von der konischen Platte (67) entfernt ist, damit die expandierbare Scheibe (30) eine nicht expandierte Ruhekonfiguration einnimmt, um eine Entriegelung der Pfanne auf der expandierbaren Scheibe (30) zu erlauben; und
- in der heruntergeklappten Stellung des Handstücks (40) die Stützfläche der expandierbaren Scheibe (30) gegen die konische Platte (67) gepresst wird, damit die expandierbare Scheibe (30) eine expandierte Konfiguration einnimmt, um eine Verriegelung der Pfanne auf der expandierbaren Scheibe (30) durch Reibung zu erlauben.

9. Hilfsvorrichtung (1) nach Anspruch 8, wobei die expandierbare Scheibe (30) in Form eines im Wesentlichen halbkugeligen Doms aus elastisch verformbarem Material vorliegt und Rillen (31) der Länge nach aufweist.

10. Set zum Implantieren von mehreren Pfannentypen für ein Hüftgelenkpfannenimplantat einer Totalhüftprothese, wobei das Set eine Hilfsvorrichtung (1) nach einem der vorangehenden Ansprüchen umfasst und mindestens ein anderes Greiforgan umfasst, das einem anderen Betätigungssystem zugeordnet ist, wobei das andere Betätigungssystem für die lösbare Anbringung auf dem Griff (2) der Hilfsvorrichtung (1) anstelle des Betätigungssystems (4) der Hilfsvorrichtung (1) geeignet ist.

## Claims

1. An ancillary (1) for placing a cup for a cotyloid implant of a hip total prosthesis, of the type including:
- a manipulating handle (2) comprising, on the one hand, a distal portion (5) designed for a manual grasping and having an impaction surface (50) and, on the other hand, a curved proximal portion (6) ending with a gripping head (60);
- a gripping member (3) adapted to cooperate with the gripping head (60) for the gripping of the cup; and
- an actuating system (4) comprising a movable grip (40) pivotally mounted according to a pivot axis (49) on the handle (2) and a mechanical transmission device (7) connecting said grip (40) to said gripping member (3), said mechanical transmission device (7) comprising a curved connecting rod (71) guided in displacement by the proximal portion (6) of the handle (2) and passing through the gripping head (60) to cooperate with said gripping member (3);
wherein, to displace the gripping member (3), the grip (40) is actuatable between:
- a deployed position so that the gripping member (3) is moved away from the gripping head (60) to allow an unlocking of the cup on the gripping member (3); and
- a folded position so that the gripping member (3) cooperates with the gripping head (60) to allow a locking of the cup on the gripping member (3);
in which the grip (40) is pivotally and removably mounted on the handle (2) and the gripping member (3) is removably fastened on the curved connecting rod (71), in order to allow the dismantling of the grip (40) and the actuating system (4) out of the handle (2),
wherein the handle (2) has a lower border (23) and an upper border (24), and the grip (40) is articulated on an indented portion (51) of said lower border (23), said indented portion (51) being delimited by two walls (52) facing each other, where the two walls (52) have notches (53) opposite to each other, and the grip (40) has, at a first end (41) articulated on the handle (2), two protruding lugs (45) received in the respective notches (53), so that, on the one hand, said lugs (45) can pivot within said notches (53) and, on the other hand, said lugs (45) can be removed out of said notches (53) when dismantling the grip (40) and the actuating system (4) out of the handle (2);
said ancillary (1) being **characterized in that** each lug (45) has a cylindrical peripheral wall truncated on two opposite sides to define two opposite flat faces (451) and two opposite arcuate faces (450), and each notch (53) is delimited by an inner peripheral wall which has an arcuate bottom (530) and two opposite flat faces (531) forming a inlet narrowed within the notch (53), where the lug (45) is introduced into the corresponding notch (53) by aligning the flat faces (451) of the lug (45) with the flat faces (531) of the notch (53) to guide the insertion of the lug (45), then the lug (45) is capable of pivoting in the notch (53) by at least one partial contact between the arcuate faces (450) of the lug (45) and the arcuate bottom (530) of the notch (53), the narrowed inlet preventing an outlet of the lug (45) out of the notch (53).

2. The ancillary (1) according to claim 1, wherein the indented portion (51) of the lower border (23), between the two walls (52) facing each other, passes through the handle (2) to the upper border (24), in order to allow the curved connecting rod (71) to pass through the handle (2), by being inserted on the side of the lower border (23), until the lugs (45) of the grip (40) are received within the notches (53) provided on the side of the lower border (23).

3. The ancillary (1) according to claim 2, wherein the upper border (24) has an elongated slot (25) extending, along the curved proximal portion (6) from the indented portion (51) opening into the upper border (24) to the gripping head (60), where the curved connecting rod (71) is displaceable at least partially within said elongated slot (25), with a translational guiding of the curved connecting rod (71) within the gripping head (60) through which it passes.

4. The ancillary (1) according to any one of the preceding claims, wherein the mechanical transmission device (7) further comprises an intermediate connecting rod (72) having two opposite ends comprising a proximal end (75) articulated on the curved connecting rod (71) according to a first pivot axis (78) and a distal end (76) articulated on the grip (40) according to a second pivot axis (77), where the intermediate connecting rod (72) has a longitudinal axis (79) which connects the first pivot axis (78) to the second pivot axis (77), and where the deployed position and the folded position are stable positions at the ends of a total tilting stroke of the grip (40) in which is included an unstable intermediate position of the grip (40) corresponding to a hard turning point towards the deployed position or towards the folded position, said intermediate position being marked by the passage of the longitudinal axis (79) of the intermediate connecting rod (72) on the pivot axis (49) of the grip (40).

5. The ancillary (1) according to claim 4, wherein the grip (40) has, at a first end (41) articulated on the handle (2), two plates (44) facing each other framing a slot (43) within which is articulated the distal end (76) of the connecting rod (72) according to a second pivot axis (77).

6. The ancillary (1) according to claim 5, wherein the lugs (45) protrude outwardly from the two respective plates (44).

7. The ancillary (1) according to any one of the preceding claims, wherein, by passing from the deployed position to the folded position, the grip (40) is pivoted towards the impaction surface (50).

8. The ancillary (1) according to any one of the preceding claims, wherein the gripping head (60) supports a conical plate (67), and the gripping member (3) comprises an expandable puck (30) having a bearing surface adapted to cooperate with the conical plate (67), so that:
- in the deployed position of the grip (40), the bearing surface of the expandable puck (30) is moved away from the conical plate (67) so that the expandable puck (30) occupies an unexpanded rest configuration to allow an unlocking of the cup on the expandable puck (30); and
- in the folded position of the grip (40), the bearing surface of the expandable puck (30) is pressed against the conical plate (67) so that the expandable puck (30) occupies an expanded configuration to allow a locking of the cup on the expandable puck (30) by friction.

9. The ancillary (1) according to claim 8, wherein the expandable puck (30) is in the form of a substantially hemispherical dome made of elastically deformable material and having grooves (31) in longitude.

10. A kit for placing several types of cups for a cotyloid implant of a hip total prosthesis, said kit comprising an ancillary (1) in accordance with any one of the preceding claims, and comprising at least one other gripping member associated with another actuating system, said other actuating system being suitable to be removably mounted on the handle (2) of said ancillary (1) instead of the actuating system (4) of said ancillary (1).
